# EUROPEAN PATENT APPLICATION

(11) **EP 4 789 559 A1**
(43) Date of publication of application: **12.08.2026**
(21) Application number: 26158007.0
(22) Date of filing: 11.02.2026
(51) Int. Cl.: A24F 40/10, A24F 40/05, A61M 11/00, A61M 15/00, A61M 11/04, A61M 15/06

(54) **VAPORIZER**

(30) Priority: 11.02.2025 LU 103534
(71) Applicant: Inspiranox Therapeutics Corporation, Dover, DE 19901 (US)
(72) Inventor: MARTINEZ GROSSO, Nicolas, Montevideo CP 11500 (UY); MOLINARI, Francisco, Cuidad de la Costa 15890 (UY)
(74) Representative: Habermann, Hruschka & Schnabel

(57) **Abstract**

The present invention relates to a vaporizer and an electronic cigarette or vape especially suited to be used for treatment of nicotine addiction and/or pulmonary conditions often associated with nicotine addiction such as chronic obstructive pulmonary disease (COPD) and pre-COPD conditions, e.g. chronic bronchitis. The vaporizer comprises:
- a vapor chamber designed to contain vapor generated from a liquid inside the chamber under temperature controlled condition;
- an air inlet designed for actively or passively introducing ambient air into the chamber under the condition that the pressure inside the chamber is lower than the ambient pressure outside the chamber;
- a vapor outlet for discharging vapor generated in the chamber and directing the vapor to a user;
- one or more means for cold vaporization of a liquid; and
- heating means for heating vapor generated in the chamber.

## Description

The present invention relates to a vaporizer and an electronic cigarette or vape especially suited to be used for treatment of nicotine addiction and/or pulmonary conditions often associated with nicotine addiction such as chronic obstructive pulmonary disease (COPD) and pre-COPD conditions, e.g. chronic bronchitis.

The concept of electric or electronic, respectively, vaporizers having a form and dimensions of a cigarette are known in the form of at least since 1930 (US 1,775,947 A, issued 16 September 1930). Gilbert (US 3,200,819 A, issued 17 August 1965) devised a smokeless non-tobacco electric cigarette.

Current electronic vaporizers for generating a vapor for inhalation in the form of a more or less cigarette-like article (also known and from time to time denoted herein as "e-cigarette" or "vape") still use a resistance coil as the heating element for generating vapor from a liquid (often referred to as "e-liquid") usually containing a base formulation composed of propylene glycol and glycerin in admixture with a variety of flavors, nicotine and other additives. The vaporization occurs in direct contact of the liquid with the resistance coil generating temperatures of at least 100 °C, typically of from 100 °C to 315 °C.

Vapes are less harmful than tobacco cigarettes because no organic parts of plants are burned. There are indications that vapes help consumers in tobacco cessation more effectively in comparison to conventional nicotine replacement therapies. However, long-term studies of potential harmful effects are missing and there are indications that the high temperature of the resistance coil leads to a contamination of the aerosol with metal particles. Furthermore, the vapor generated from conventional e-liquids is often perceived as unpleasant by consumers, especially those who want to quit smoking, reducing compliance rate. Moreover, the high temperatures occurring in vaporization by conventional vapes make them incompatible with application of drugs such as active ingredients helping nicotine-addicted customers in the process of tobacco cessation or drugs used for treatment of condition often associated with nicotine addiction, in particular COPD.

The problem underlying the present invention is to provide a novel vape system overcoming one or more of the deficiencies of prior art vaporizers.

The solution to the above technical problem is provided by the present invention as characterized in the claims and further disclosed in the present disclosure and drawings.

In particular, the present invention provides a vaporizer comprising
- a vapor chamber designed to contain vapor generated from a liquid inside the chamber under temperature controlled condition;
- an air inlet designed for actively or passively introducing ambient air into the chamber under the condition that the pressure inside the chamber is lower than the ambient pressure outside the chamber;
- a vapor outlet for discharging vapor generated in the chamber and directing the vapor to a user;
- one or more means for cold vaporization of a liquid; and
- heating means for heating vapor generated in the chamber.

In certain instances of the present disclosure the term "vapor" is also denoted as "fog" and these terms may be used interchangeably for describing the aerosol generated from a liquid inside the vaporizer by the means for cold vaporization of the liquid.

The person skilled in the art understands that the chamber is, in some embodiments except for the air inlet and vapor outlet, reversibly sealed against the outer environment, in particular the chamber is reversibly air (i.e. gas) and liquid tight against the outer environment. The terms "reversibly sealed", "reversibly airtight", "reversibly gas tight", "reversibly liquid tight" mean that the chamber can be opened and closed as convenient, e.g. for cleaning or maintenance purposes or for introducing the liquid to be vaporized, and closed thereafter, and when it is in its closed, the chamber is, in some embodiments except for the air inlet and vapor outlet, sealed from the outer environment, in particular airtight (gastight) and liquid tight.

According to the invention, the air inlet is designed such that ambient air is introduced into the chamber when the pressure in the chamber is lower than the ambient pressure outside the chamber. The air inlet may be designed such that the air is introduced actively into the chamber under said condition. This may be embodied by an air transportation system combined with pressure sensors measuring the ambient pressure and the pressure in the vapor chamber in combination with means comparing the pressure measured by the sensor outside the chamber with the pressure measured inside the chamber such that, when the pressure measured outside the chamber is greater than the pressure measured inside the chamber, the air transportation system causes air to enter the chamber as long as the pressure inside the chamber reaches the pressure outside the chamber. The air transportation system can be embodied e.g., by pumping means or suction means. In other embodiments of the invention ambient air is introduced passively when the ambient pressure outside the chamber is greater than the pressure inside the chamber, preferably by a one-way valve that opens exclusively when the ambient pressure outside the chamber is greater than the pressure inside the chamber.

In preferred embodiments of the invention, the vaporizer further comprises means for reversibly closing the vapor outlet, preferably when the vaporizer is not in use by a user. The means for reversibly closing the vapor outlet can be e.g., a one-way valve provided in the lumen of the vapor outlet exclusively when the user sucks on the vaper outlet. In other embodiments, the vaper outlet can be reversibly closed by a closing mechanism covering the outside opening of vaper outlet.

The term "cold vaporization" means that the liquid is vaporized essentially without the use of heat elements. Preferred means for cold vaporization of the liquid are means for emitting ultrasonic waves into the liquid such as an ultrasonic head which is also synonymously referred to herein as "ultrasonic element". In preferred embodiments of the invention, the ultrasonic head comprises a membrane for generating ultrasonic waves for vaporization of the liquid. In other preferred embodiments, the ultrasonic head comprises one or more reverse piezoelectric elements (i.e. excited crystals generate ultrasonic waves due to reverse piezoelectric effect) generating ultrasonic waves causing vaporization of the liquid. Vaporization by means of reverse piezoelectric elements is especially useful when included as means for cold vaporization in the inventive e-cigarette since the reverse piezoelectric elements are highly suited for miniaturization.

It is preferred according to the invention that the liquid is in direct contact with the ultrasonic waves-emitting element, preferably the ultrasonic head.

Cold vaporization, in particular by ultrasonic waves emitted from an ultrasonic elements leads to a relatively cold vapor which can be problematic for the user inhaling the cold vapor which frequently leads at least to an uncomfortable feeling (and, thus, to low user friendliness and low user acceptance of the vaporizer), but may in worser case also lead to bronchial constriction which would be highly problematic for users suffering from pulmonary conditions. In particular, bronchial constriction would be a serious problem for subjects having COPD or pre-COPD conditions such as chronic bronchitis.

To circumvent the above-described problems, the vaporizer of the invention comprises means for heating vapor generated in the chamber are attached to at least a part of the wall(s) of the chamber or integrated in at least a part of the wall(s) of the chamber. The heating means preferably are designed and, more particularly, regulated such that the vapor is heated to a temperature of about less than 100 °C, preferably from about ambient temperature such as about 20, about 21, about 22, about 24 or about 25 °C to about less than 90 °C, more preferably about 30 to about 80 °C, most preferably about 30 °C to about 40 °C.

In preferred embodiments, the chamber of the vaporizer according to the present invention contains a liquid to be vaporized by the means for cold vaporization. Preferably, the liquid is water or an aqueous solution, suspension or emulsion. In preferred embodiments, the liquid, preferably the aqueous solution, suspension or emulsion, comprises one or more additives such as nicotine and/or one or more drugs and/or one or more flavors. Preferred additives are selected, for example, from bactericidal and virucidal drugs, salts such as NaCl, KCl,, LiCl and mixtures thereof, amino acids such as arginine, and buffering agents such as NaHCO₃. In certain embodiments of the invention the composition comprises a stimulant. A preferred stimulant is nicotine. Further preferred additives for use in the invention include, but are not limited to, non-steroidal anti-inflammatory drugs (NSAIDs) such as ibuprofen, naproxen, flurbiprofen, ketoprofen, diclofenac, diflunisal, etodolac, fenoprofen, indomethacin, meclofenamate, mefenamic acid, meloxicam, nabumetone, oxaprozin, piroxicam, sulindac, tolmetin, celecoxib, acetylated salicylates, non-acetylated salicylates, and combinations thereof. Especially preferred NSAIDs for use in the invention are ibuprofen and naproxen and mixtures thereof, most preferably ibuprofen.

Especially preferred liquid compositions for use in the present invention are hypertonic solutions of NSAIDs, such as compositions disclosed in US 2018/0296509 A1, the contents of which are hereby incorporated in its entirety by reference, such as described in paragraph [0023] thereof.

Particularly preferred compositions for use in the invention are bactericidal and virucidal compositions characterized by comprising a NSAID at a concentration of from about 5 and to about 500 mM solubilized in saline solution, preferably a hypertonic saline solution comprising a concentration of from about 0.3 to about 2 Molar NaCl; more preferably from about 0.4 and 1.1 M NaCl; even more preferably from about 0.9 to about 1.05 M NaCl. More preferably, the concentration of NSAID is in the range from about 5 to about 180 mM; more preferably less than about 180 mM; even more preferably from about 5 to about 50 mM. Preferably, the NSAID is selected from those as outlined above (including mixtures thereof), most preferably ibuprofen. Preferred counterions are monovalent cations selected from sodium, potassium, lithium and combinations thereof. Preferably, aqueous compositions, in particular aqueous solutions, for use in the invention have pH of from about 6.0 to about 8.5; preferably from about 7.0 to about 8.0; more preferably from about 7.5 to aobut 7.9.

In preferred embodiments, the vaporizer according to the invention comprises one or more reservoirs designed to contain one or more substances (also denoted herein as "additives") to be introduced into the chamber, the reservoir having a reversibly closeable connection to the chamber. In one embodiment, the reservoir is designed to contain the liquid to be vaporized in the chamber. A reservoir designed for containing the liquid is also denoted herein as "liquid reservoir". In other embodiments, at least one reservoir is designed to contain one or more additives such as nicotine and/or drugs and/or flavors to be introduced in the chamber. A reservoir designed for containing additive(s) is also denoted herein as "additive reservoir". Preferably, an additive reservoir is arranged in the vaporizer such that the additive(s) are introduced into the vapor generated in the chamber. In other embodiments, the vaporizer may comprise (instead of a reservoir or additionally thereto) an access for introducing one or more substances into the vapor chamber. An access may be provided as an reversibly closable connection into the chamber such as by screw mechanism.

In certain embodiments of the invention the vaporizer further comprising means for hindering liquid droplets from entering the vapor outlet.

The vaporizer of the invention preferably further comprises means for supplying the cold vaporization means with electric current. The electric current supplying means are preferably embodied by a battery or accumulator, preferably a rechargeable battery or rechargeable accumulator. The vaporizer of the invention preferably comprises means for controlling the means for cold vaporization of a liquid, preferably the one or more ultrasonic heads.

The vaporizer of the invention may preferably be provided in the form of and having the dimensions of an electric cigarette.

Thuss, the present invention also relates to an e-cigarette comprising at least the essential components of the vaporizer as defined herein. Preferred embodiments of the e-cigarette according to the invention have already been described above.

The invention also relates to a method of treatment of COPD or a pre-COPD condition such as chronic bronchitis in a subject in need comprising administration of a vapor generated from a liquid as defined herein by means of the vaporizer and/or e-cigarette of the invention.

The present invention is further described with reference to drawings showing schematic representations of embodiments of the vaporizer of the invention.

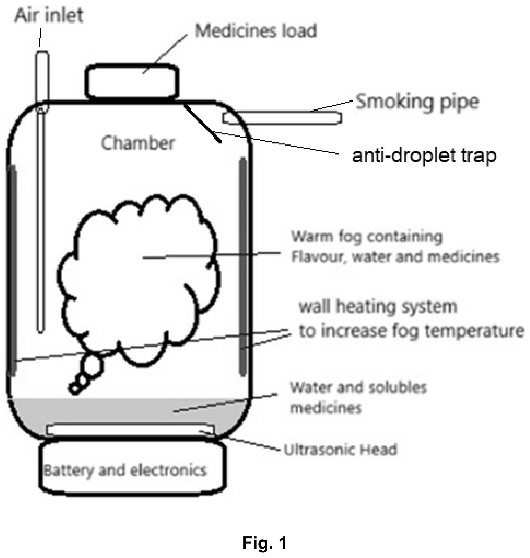

Fig. 1 shows a schematic cross section of an exemplary vaporizer of the invention. The vaporizer of Fig. 1 comprises a central chamber containing an amount of liquid in the lower part. An ultrasonic head is arranged at the bottom of the chamber and reaches into the liquid. In the exemplary embodiment of Fig. 1 the liquid is water containing soluble medicines. Below the ultrasonic head and provided as a socket for the chamber is arranged a battery for supplying electric current to the ultrasonic head and further comprising electronic control elements. Applied at least to parts of the chamber walls is a heating system for increasing the temperature of vapor generated by means of the ultrasonic head in the chamber above the liquid. The upper or top part of the chamber comprises a vapor outlet, denoted in Fig. 1 as smoking pipe, through which the heated vapor generated in the chamber can be directed to a user who will typically puff on the smoking pipe to remove an amount of vapor thereby introducing the vapor into the user's pulmonary system. An air inlet for supplying the chamber with ambient air for pressure equalization when the vapor is generated and removed from the chamber is arranged at the top of the chamber. The air inlet is typically an elongate tube reaching into the chamber to a position in proximity to the liquid for avoiding that air entering the chamber via the air inlet is short circuit to the vapor outlet. An screen functioning as an anti-drop trap is provided in the vicinity of the vapor outlet to prevent larger droplets from entering the vapor outlet. A reservoir is provided on the top side of the chamber for containing medicines to be introduced in the chamber, either into vapor or into the liquid, for administering such medicines via the vapor to the user.

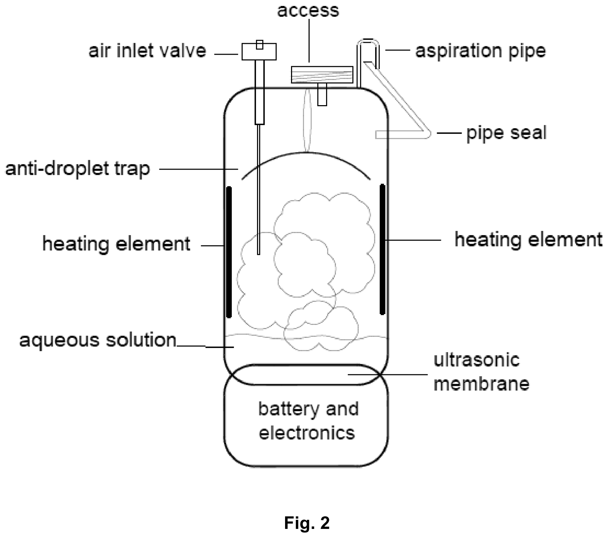

Fig. 2 shows a schematic cross section of a further exemplary vaporizer of the invention. The vaporizer of Fig. 2 comprises a central chamber containing an amount of liquid, in the case of Fig. 2 an aqueous solution containing, e.g. salt(s), buffering agent(s), drug(s) such as one or more NSAIDs and/or nicotine, in the lower part. An ultrasonic head comprising an ultrasonic membrane is arranged at the bottom of the chamber. The ultrasonic membrane is in contact with the aqueous solution to be vaporized. Below the ultrasonic head and provided as a socket for the chamber is arranged a battery for supplying electric current to the ultrasonic head and further comprising electronic control elements. Applied at least to parts of the chamber walls are heating elements, e.g. in case the chamber has the form of a cylinder, the heating elements may be circumferentially provided in a section of the chamber wall, for increasing the temperature of vapor generated by means of the ultrasonic membrane in the chamber above the aqueous solution. The upper or top part of the chamber comprises a vapor outlet, denoted in Fig. 2 as aspiration pipe, through which the heated vapor generated in the chamber can be directed to a user who will typically puff (or suck) on the aspiration pipe to remove an amount of vapor thereby introducing the vapor into the user's pulmonary system. An air inlet for supplying the chamber with ambient air for pressure equalization when the vapor is generated and removed from the chamber is arranged at the top of the chamber and comprises an air inlet valve which opens only when the pressure inside the vapor chamber is lower than the ambient pressure outside the vapor chamber. The air inlet is typically an elongate tube reaching into the chamber to a position in proximity to the liquid for avoiding that air entering the chamber via the air inlet is short circuit to the vapor outlet. A screen functioning as an anti-droplet trap is provided as a screen in the in the upper part of the chamber in form of a central screen below the vapor outlet to prevent larger droplets from entering the vapor outlet. An access element is provided on the top side of the chamber for reversibly closing the chamber which enables that medicines and/or other additives can be introduced into the chamber, either into vapor or into the liquid, for administering such medicines and/or other additives via the vapor to the user. Once the medicines and/or additives have been introduced into the chamber the access is closed by a typical closing mechanism such as a screw.

## Claims

1. A vaporizer comprising
- a vapor chamber designed to contain vapor generated from a liquid inside the chamber under temperature controlled condition;
- an air inlet designed for actively or passively introducing ambient air into the chamber under the condition that the pressure inside the chamber is lower than the ambient pressure outside the chamber;
- a vapor outlet for discharging vapor generated in the chamber and directing the vapor to a user;
- one or more means for cold vaporization of a liquid; and
- heating means for heating vapor generated in the chamber.

2. The vaporizer of claim 1 wherein the means for cold vaporization of a liquid is one or more ultrasonic head.

3. The vaporizer of claim 1 or 2 wherein the means for heating vapor generated in the chamber are attached to at least a part of the wall(s) of the chamber or integrated in at least a part of the wall(s) of the chamber.

4. The vaporizer according to any one of the preceding claims wherein the air inlet comprises a one way valve exclusively opening when the pressure inside the chamber is lower than the ambient pressure outside the chamber.

5. The vaporizer according to any one of the preceding claims further comprising means for reversibly closing the vapor outlet.

6. The vaporizer according to any one of the preceding claims wherein the chamber contains a liquid to be vaporized by the means for vaporization.

7. The vaporizer of claim 6 wherein the liquid is water or an aqueous solution.

8. The vaporizer of claim 6 or 7 wherein the liquid comprises one or more additives.

9. The vaporizer of claim 7 or 8 wherein the additive(s) is/are selected from nicotine, drugs, salts, buffering agents and flavors.

10. The vaporizer according to any one of the preceding claims further comprising a reservoir designed to contain a substance or substances to be introduced into the chamber, the reservoir having a reversibly closable connection to the chamber.

11. The vaporizer according to any one of the preceding claims further comprising means for hindering liquid droplets from entering the vapor outlet.

12. The vaporizer according to any one of the preceding claims provided in the form of and having the dimensions of an electronic cigarette.
